# EUROPEAN PATENT APPLICATION

(11) **EP 3 586 747 A1**
(43) Date of publication of application: **01.01.2020**
(21) Application number: 18179211.0
(22) Date of filing: 22.06.2018
(51) Int. Cl.: A61B 6/00, A61B 6/03

(54) **PLANNING A PROCEDURE FOR CONTRAST IMAGING OF A PATIENT**

(71) Applicant: Koninklijke Philips N.V., 5656 AE Eindhoven (NL)
(72) Inventor: BIPPUS, Rolf Dieter, 5656 AE Eindhoven (NL); WIEGERT, Jens, 5656 AE Eindhoven (NL); BYSTROV, Daniel, 5656 AE Eindhoven (NL)
(74) Representative: de Haan, Poul Erik

(57) **Abstract**

The invention refers to a system for planning a procedure for contrast imaging. A contrast agent estimation unit 6 estimates an amount of contrast agent, which would be present in tissue represented by a voxel of a planning image, for instance a CT image, during contrast imaging, based on characteristics of the tissue and the contrast agent. An attenuation image generating unit 7 generates an attenuation image based on the planning image and the estimated amount of contrast agent, and a procedure planning unit 8 plans the procedure based on the attenuation image. Thus, the planning of the procedure can be based on the attenuation image showing for each patient an expected image resulting from contrast imaging. Accordingly, the planning of the administration of the contrast agent and/or the imaging settings, can be based on reliable and detailed data such that the planning for an individual patient becomes more effective.

## Description

### FIELD OF THE INVENTION

The invention relates to a system, a method and a computer program for planning a procedure for contrast imaging of a patient.

### BACKGROUND OF THE INVENTION

When a procedure for contrast imaging of a patient is planned, for instance, when the administration of a contrast agent or a tube current of a CT imaging device for such a procedure is planned, individual CT planning scans, i.e. scout scans, are acquired in which the contrast agent is not present. Thus, for planning the exact dose, administration protocol or current setting, a user, i.e. a physician, has to rely on his/her experience when considering the contrast that might be achieved by a specific contrast agent dose or current setting based on the planning scan. Thus, the planning of the administration of the contrast agent is limited in its effectiveness and it is difficult to provide a patient specific procedure for contrast imaging for each individual patient.

### SUMMARY OF THE INVENTION

It is an object of the present invention to provide a system, a method and a computer program for planning a procedure for contrast imaging of a patient which allow for a very effective generation of a procedure for contrast imaging for each individual patient. In a first aspect of the present invention a system for planning a procedure for contrast imaging of a patient is presented, wherein the system comprises
- a planning image providing unit for providing a planning image of a region of interest of the patient,
- a contrast agent estimation unit for estimating for each voxel of the planning image an amount of contrast agent, which would be present in tissue represented by the respective voxel of the planning image during contrast imaging, based on characteristics of the tissue represented by the respective voxel and on characteristics of the contrast agent,
- an attenuation image generating unit for generating an attenuation image based on the planning image and the estimated amount of contrast agent for each voxel, wherein the attenuation image is indicative of the attenuation caused by the contrast agent in the region of interest during contrast imaging,
- a procedure planning unit for planning a procedure for contrast imaging of the patient based on the attenuation image.

Since the contrast agent estimation unit estimates an amount of contrast agent for each voxel of the planning image that would be present in tissue represented by the respective voxel during contrast imaging based on characteristics of tissue and on characteristics of the contrast agent and since the attenuation image generation unit uses the estimation of the amount of the contrast agent to generate an attenuation image, the procedure planning unit can base the planning of the procedure for contrast imaging on the attenuation image showing in detail for each individual patient an expected image resulting from contrast imaging. Accordingly, the planning of the procedure for contrast imaging, i.e. the planning of the administration of the contrast agent and/or the imaging settings, can be based on reliable and detailed data of an individual patient. Thus, the planning of the procedure for an individual patient becomes more effective.

The procedure for contrast imaging refers to any procedure in which during a medical imaging a certain part of the body of the patient imaged is enhanced by applying a contrast agent, i.e. a substance that comprises an attenuation coefficient that increases the visibility of a region comprising the substance in a medical image. The planning image providing unit provides a planning image of a region of interest of a subject, for instance, by providing a CT image of a region of interest of the patient being previously acquired. The planning image can be any image of the patient that can be used for planning the contrast imaging procedure. Preferably, the planning image is a 3D scout scan image that can be acquired fast and with a low radiation dose. More preferably the scout scan image is a 3D scout scan image acquired using a radiation dose that is in the range of current standard 2-dimensional surview acquisitions, i.e. in a range between 0.8 and 3.0 mAs. Thus, during the scout scan only a negligible dose is provided to the patient. For instance, a CT scanner allowing the acquisition of sparse views can be used for acquiring such a scout scan.

The contrast agent estimation unit estimates the amount of contrast agent for each voxel of the planning image based on the characteristics of the tissue represented by the voxel, wherein the tissue represented by the voxel is determined, for instance, using a known tissue classification provided by a user or a neural network. In a preferred embodiment the tissue represented by a voxel is determined by segmenting the planning image. Based on the kind of tissue, for instance liver tissue or lung tissue, the contrast agent estimation unit estimates how much contrast agent can be expected to be present in the tissue represented by the voxel after administration of a contrast agent using further knowledge of the characteristics of the preferred contrast agent. The characteristics of tissue and of the contrast agent can be stored in a library provided on a storage unit. The characteristics can comprise, for instance, concentration-time-curves expected for a contrast agent in a certain tissue. Moreover, the attenuation image generating unit can use a numerical model simulating an expected amount of contrast agent in each voxel of the planning image based on the stored characteristics of the contrast agent and characteristics of the tissue represented by the voxel. Furthermore, the contrast agent estimation unit can use a database in which a plurality of planning images with a known amount of contrast agent for each voxel are stored and compare the planning image with the stored planning images to determine the amount of contrast agent. Moreover, in a preferred embodiment the contrast agent estimation unit and optionally the attenuation image generation unit can comprise a neural network, wherein the database can be used for training the neural network such that the neural network is able to provide a map of the estimated amount of contrast agent or optionally directly the attenuation image. The map provided by the neural network can be indicative for the estimated amount of contrast agent for all voxels of the planning image that was provided to the neural network as input.

The attenuation image generation unit generates an attenuation image based on the planning image and the estimated amount of contrast agent for each voxel, for instance, the attenuation image generation unit uses the estimated amount of contrast agent in a voxel to determine an expected amount of attenuation of this voxel when the contrast agent is present in the tissue represented by the voxel and changes the attenuation, i.e. the grey value, of the planning image accordingly to generate the attenuation image. Thus, the attenuation image is indicative of the attenuation caused by the contrast agent in the region of interest. The procedure planning unit plans the procedure for contrast imaging of the patient based on the attenuation image, i.e. uses known planning methods and algorithms for planning, for instance, an administration protocol and dose of the contrast agent and/or an imaging setting for the imaging unit used for the contrast imaging, wherein the attenuation image is used as input.

In an embodiment the contrast agent estimation unit is further adapted to estimate the amount of contrast agent, which would be present in tissue represented by the respective voxel of the planning image during contrast imaging, based on a predetermined administration protocol of the contrast agent and/or a predetermined imaging setting of an imaging unit used for contrast imaging. In this embodiment, the estimate of the amount of contrast agent is not only based on the characteristics of the tissue and the characteristics of the contrast agent, but can be further based on a predetermined administration protocol of the contrast agent or on further characteristics, i.e. imaging settings, of a predetermined imaging unit used for contrast imaging. The predetermined administration protocol can be, for instance, a standard protocol, wherein the procedure planning unit is adapted to modify the standard protocol based on the generated attenuation image. Moreover, the predetermined imaging settings can be standard imaging settings for the imaging unit, wherein the procedure planning unit is adapted to modify the standard imaging settings based on the generated attenuation image. For instance, if with regard to the standard protocol or standard imaging setting the attenuation of specific parts of the region of interest is not high enough, the procedure planning unit can be adapted to increase the amount of administered contrast agent with reference to the standard protocol or increase a current setting for the imaging unit with reference to the standard setting. Alternatively, the predetermined administration protocol or imaging setting can be an administration protocol or imaging setting preferred by the user, i.e. a physician, and the procedure planning unit can be adapted to determine based on the attenuation image if this preferred administration protocol or imaging setting leads to the expected results.

Further, when the planning image is used for planning imaging settings of an imaging unit planned to be used during contrast imaging a predetermined contrast agent and a predetermined application protocol can be used to determine the amount of contrast image for each voxel.

In an embodiment the attenuation image generating unit is adapted to determine an estimate of an attenuation caused by the contrast agent in tissue represented by a voxel based on the estimated amount of contrast agent in tissue represented by the voxel, wherein for generating the attenuation image the attenuation image generating unit is adapted to add the estimate of the attenuation caused by the contrast agent for each voxel to the planning image. The attenuation caused by the contrast agent in tissue represented by a voxel is indicated by an attenuation value, i.e. grey value, of the voxel. Preferably, a mathematical relationship between an amount of contrast agent and the attenuation caused by the contrast agent in tissue in an image of the region of interest is stored in a storage unit, wherein the attenuation image generating unit uses this mathematical relationship to determine an estimate of an attenuation caused by the contrast agent for each voxel, i.e. to determine an attenuation value for each voxel. Moreover, a mathematical relationship can be used that directly determines an attenuation value for a voxel based on the estimated amount of contrast agent.

Preferably, the attenuation image generating unit is adapted to determine the estimate of an attenuation caused by the contrast agent for a voxel based on a predetermined linear relationship between an amount of contrast agent in tissue and the attenuation caused by the contrast agent. In this embodiment a known attenuation coefficient of the contrast agent at 100% concentration can be linearly rescaled with the estimated concentration of the contrast agent, i.e. the estimated amount of the contrast agent. This mathematical relationship is especially suitable when an imaging unit that should be used for the contrast imaging procedure is a non-spectral CT system.

Further, it is preferred that the attenuation image generating unit is adapted to determine the estimate of an attenuation caused by the contrast agent with respect to radiation energy provided by an imaging unit planned to be used during contrast imaging or with respect to contributions of photon and Compton scattering to the attenuation. Taking the energy provided by the imaging unit or the contributions of photon and Compton scattering into account is especially advantageous if the imaging unit that should be used for the contrast imaging procedure is a spectral CT system and/or if the contrast agent that should be used comprises an attenuation coefficient showing a K-edge, for instance, iodine. Moreover, a model can be provided for modelling the characteristics of a specific imaging unit using predetermined imaging settings, like the imaging settings used in a diagnostic image, to simulate based on the amount of contrast agent in each voxel the attenuation that would be expected in a voxel in accordance with the imaging unit and the imaging settings. The model can be adapted to take the attenuation caused by a contrast agent with respect to different radiation energies into account.

In an embodiment the system further comprises a segmentation providing unit for providing a segmentation of the planning image into a plurality of segments, wherein the contrast agent estimation unit estimates the amount of contrast agent present in tissue represented by a voxel belonging to a segment based on characteristics of tissue within the segment. The segmentation unit is adapted to determine the different segments of the region of interest, for instance, based on the different tissue types present in the region of interest, wherein the different tissue types of the segments can be determined based on, for instance, a mean attenuation value of the segment, i.e. based on a mean grey value, or based on the structure or outline of the segment. Since the planning image is segmented and the amount of contrast agent for each voxel determined based on the characteristics of tissue within the segment to which the respective voxel belongs, the tissue represented by the voxel can be determined based on more parameters than the attenuation of the voxel and/or its neighbors. For instance, the tissue can also be determined based on the structure, form and/or location of the segment. Thus, the tissue represented by a voxel and therefore also the characteristics of tissue that should be used when estimating the amount of contrast agent can be determined more accurately.

Preferably, the segmentation unit is adapted to determine a probability that a voxel of the planning image represents a specific organ or a specific anatomical region, wherein the segmentation is further adapted to provide the segmentation of the planning image based on the probability. The probability that a voxel belongs to a specific organ or a specific anatomical region can be determined based on known segmentation methods, for instance, based on the attenuation of the voxel, i.e. the grey value of the voxel, and upon the attenuation values of the voxels surrounding the voxel. In an embodiment the probability of the voxel representing a certain tissue can only be 1 or 0, wherein 1 indicates that the voxel represents the tissue and 0 indicates that the voxel does not represent the tissue. In an alternative embodiment also other values than 0 and 1 can be used as probability for a voxel. In an embodiment the segmentation can be based in a neural network as disclosed, for instance, in the article "Foveal fully convolutional nets for multi-organ segmentation" by T. Brosch et al., Proceedings of SPIE 10574, Medical Imaging 2018: Image Processing (2018).

Moreover, it is preferred that the segmentation unit is adapted to segment the planning image based on a predetermined anatomical atlas or based on a predetermined organ model. Using an anatomical atlas or a predetermined organ model can lead to a very fast and reliable segmentation even in planning images that have a low resolution or a very low contrast. The contrast agent estimation unit that estimates the amount of contrast agent based on the characteristics of the tissue within the segments, i.e. the kind of tissue in the segments, is not only determined based on the attenuation of the voxel itself, but is also determined by the surrounding voxels, i.e. by the segment to which the voxel belongs. Accordingly, the determination of the amount of contrast agent becomes more reliable and less prone to faulty attenuation values in the planning image.

In an embodiment the procedure planning unit is adapted for planning imaging settings of an imaging unit planned to be used during the procedure for contrast imaging based on the attenuation image.

Further in an embodiment the planning image provided by the planning image providing unit has been acquired with the same imaging unit planned to be used during the procedure for contrast imaging. Preferably, the imaging unit for contrast imaging is a CT imaging unit. More preferably the imaging unit is a spectral CT imaging unit.

Preferably, the planning image is a 3D scout scan image. Moreover, it is preferred that the planning image provided by the planning image providing unit has been acquired using a CT-system with a grid-switching x-ray tube for providing the planning image. Furthermore, it is preferred that the planning image provided by the planning image providing unit has been acquired using a sparse angular sampling acquisition. For instance, a CT-system with grid-switching ability allows for the sparse angular sampling acquisition. If a sparse angular sampling acquisition is used, an appropriate 3D scout scan can be acquired using relatively few views compared to the views necessary for a full CT image. Since only very few views are necessary, the views can be acquired at a relatively high tube current, while keeping the overall dose small. Thus, scout images with less noise and photon starvation and therefore with a higher image quality compared to a scout image acquired with a standard acquisition protocol at comparable radiation dose can be provided.

In a further aspect of the present invention, a method for planning a procedure for contrast imaging of a patient is presented, wherein the method comprises the steps of:
- providing a planning image of a region of interest of the patient,
- estimating for each voxel of the planning image an amount of contrast agent, which would be present in tissue represented by the respective voxel of the planning image during contrast imaging, based on characteristics of the tissue represented by the respective voxel and on characteristics of the contrast agent,
- generating an attenuation image based on the planning image and the estimated amount of contrast agent for each voxel, wherein the attenuation image is indicative of the attenuation caused by the contrast agent in the region of interest during contrast imaging,
- planning a procedure for contrast imaging of the patient based on the attenuation image.

In a further aspect of the present invention, a computer program for planning a procedure for contrast imaging of a patient is presented, wherein the computer program comprises program code means for causing the system of claim 1 to carry out the steps of the method as defined in claim 14 when the computer program is run on a computer controlling the system.

It shall be understood that the system of claim 1, the method of claim 14 and the computer program of claim 15 for planning a procedure for contrast imaging of a patient have similar and/or identical preferred embodiments, in particular as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereafter.

### BRIEF DESCRIPTION OF THE DRAWINGS

In the following drawings:
Fig. 1 shows schematically and exemplarily an embodiment of a system for planning a procedure for contrast imaging of a patient,
Fig. 2 shows an example for the images generated by the system, and
Fig. 3 shows a flowchart exemplarily illustrating an embodiment of a method for planning a procedure for contrast imaging of a patient.

### DETAILED DESCRIPTION OF EMBODIMENTS

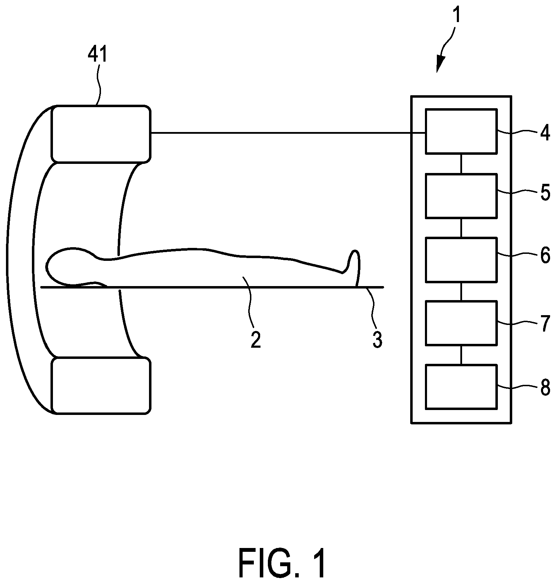

Fig. 1 shows schematically and exemplarily an embodiment of a system for planning a procedure for contrast imaging of a patient. In this embodiment the system 1 is adapted for planning imaging settings during the procedure for a patient 2 lying on a support means 3 like a patient table. The system comprises a planning image providing unit 4 for providing a planning image of a region of interest of the patient, for instance, of a specific organ of the patient. In this embodiment the planning image providing unit 4 is connected to a CT imaging system 41 with a grid switching x-ray tube. The planning image is acquired by the CT imaging system 41 adapted to provide a sparse angular sampling acquisition. The planning image providing unit 4 is in this embodiment adapted to reconstruct a 3D scout scan image as planning image from imaging data provided by the CT imaging system 41. In other embodiments the planning image can be an image acquired during a previous diagnostic scan or can be a planning image previously acquired and stored in a storage unit that may be a part of the planning image providing unit 4.

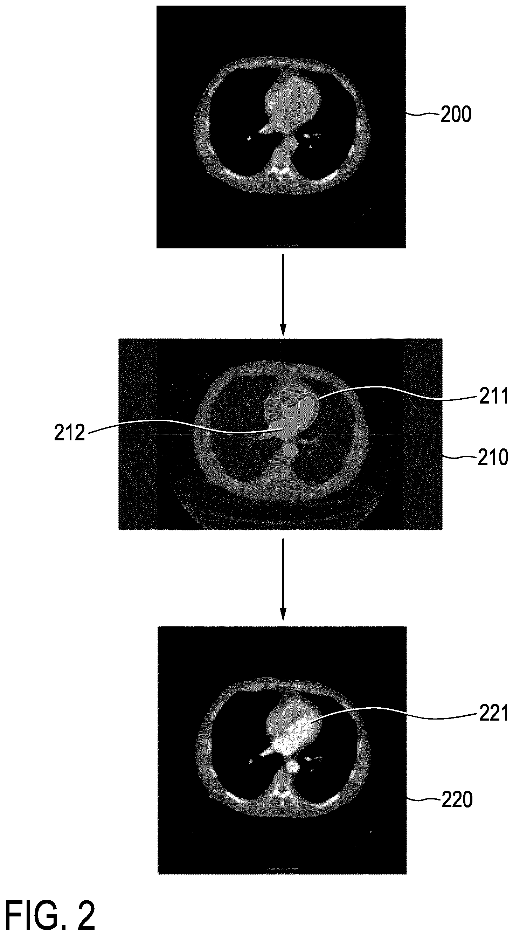

The system 1 further comprises a segmentation unit 5, a contrast agent estimation unit 6, an attenuation image generating unit 7 and a procedure planning unit 8. The operation of these units of the system 1 will be further explained also with reference to Fig. 2. The planning image providing unit 4 provides as planning image the 3D scout scan image. The image 200 shown in Fig. 2 represents an example of a 2D sectional view of a 3D scout image as can be provided by the image providing unit 4. The segmentation unit is adapted to use a known segmentation algorithm, for instance, based on a predetermined anatomical atlas of the region of interest, to segment the planning image 200 into a plurality of tissue segments 211. The segments provided by the segmentation unit 5 comprise knowledge of the tissue in the provided segments 211. Thus, contrast agent estimation unit 6 can be adapted to determine the kind of tissue within each segment, for instance, based on the attenuation of the voxels within the segment or based on the form, structure or location of the segment and/or based on labels of an anatomical atlas used for segmentation. Accordingly, contrast agent estimation unit 6 can determine for each voxel in a segment which tissue the voxel represents. Moreover, a user can provide a preferred contrast agent as input to the contrast agent estimation unit 6, wherein the contrast agent estimation unit 6 determines relevant characteristics of the contrast agent from a library storing a plurality of contrast agents and their characteristics. Alternatively, the contrast agent estimation unit 6 can use a standard contrast agent as default and/or the user can input the specific characteristics of the contrast agent. Further, the user can input a preferred administration protocol for the contrast agent. Alternatively, a standard administration protocol can be used. The contrast agent estimation unit 6 is adapted to use the knowledge about the tissue that is represented by each segment, i.e. voxel, and also the knowledge on the characteristics of the contrast agent to estimate the amount of contrast agent that is expected after administration of the contrast agent in each segment, i.e. voxel of the segment. Moreover, the contrast agent estimation unit 6 can additionally use the knowledge about the administration protocol, if this knowledge is provided. Preferably, a database is provided containing a default estimate of the amount of contrast agent for a voxel based on the characteristics of the tissue, for instance, the kind of tissue, and the characteristics of a contrast agent, for instance, the kind of contrast agent. Alternatively, other methods might be used, for instance, the contrast agent estimation unit 6 can be adapted to provide a time-concentration-curve for the known combination of a contrast agent and the characteristics of a tissue, for instance, the kind of tissue and an expected blood flow through the tissue, and determine the expected amount of contrast agent based on the time-concentration-curve. Further, for determining the amount of contrast agent a data base of patient images acquired during a contrast imaging procedure with a specific contrast agent and/or a specific administration protocol can be used. In this embodiment a neural network approach using the 3D scout scan as input and the database as training data is preferred.

Based on the estimated amount of contrast agent the attenuation image generation unit 7 determines an estimate of the attenuation caused by the contrast agent in each segment during contrast imaging, i.e. estimates an attenuation value or grey value for each voxel indicative for the attenuation of the contrast agent, generating, for instance, image 210 as shown in Fig. 2. Image 210 shows the segmented areas 211 provided by the segmentation unit 5 and the estimated increased attenuation caused by the contrast agent in certain segments, for instance, in segment 212. The attenuation of the contrast agent can be estimated, for instance, based on a mathematical relationship between the amount of contrast agent and the attenuation caused by the contrast agent for a specific imaging modality and/or specific imaging settings, for instance, for a CT imaging system and predetermined diagnostic imaging settings. In the here-described embodiment a linear relationship between the estimated amount of contrast agent and attenuation caused by the contrast agent is assumed, such that an attenuation coefficient of the contrast agent is linearly rescaled based on the estimated amount of contrast agent. The attenuation image generation unit 7 uses image 210 as a basis for generating an attenuation image 220. In this embodiment the attenuation image generation unit 7 adds the image 210, i.e. the estimated attenuation value for each voxel, to the attenuation values of planning image 200 to generate the attenuation image 220 showing expected attenuation values 221, i.e. grey values, after application of a contrast agent. The procedure planning unit 8 then uses known planning algorithms for planning the imaging settings of the imaging device during the contrast imaging procedure based on the attenuation image 220. Preferably, the procedure planning unit 8 is adapted to plan the optimal radiation dose and the optimal tube current settings for the contrast imaging procedure. For instance, the user can determine a preferred contrast between the tissue of interest and/or a predetermined tissue. The procedure planning unit 8 can then, for instance, use standard or default imaging setting for determining an expected contrast between the tissue of interest and surrounding tissue and/or a predetermined tissue based on the attenuation image. Based on the determined contrast the procedure planning unit 8 can adapt the standard or default imaging settings until the preferred contrast can be expected during the contrast imaging procedure. For determining the expected contrast a model of the imaging system can be provided that allows the estimation of tissue contrasts based on the attenuation image. Other examples for determining imaging settings based on a planning image can be found, for instance, in the articles "Dose reduction in CT by anatomically adapted tube current modulation. I. Simulation studies" by M. Giess et al., Medical Physics, volume 26, pages 2235 - 2247 (1999), "Computer-Assisted Scan Protocol and Reconstruction (CASPAR) - Reduction of Image Noise and Patient Dose", by J. Sperl et al., IEEE Transaction on Medical Imaging, volume 29, pages 724 - 732 (2010) and "Patient specific tube current modulation for CT dose reduction" by Y. Jin et al., Proceedings of SPIE 9412, Medical Imaging 2015: Physics of Medical Imaging (2015).

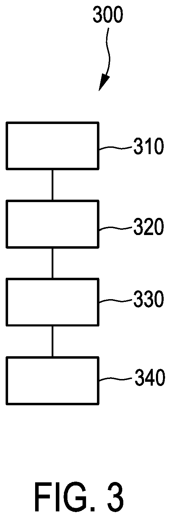

Fig. 3 shows a flowchart exemplarily illustrating an embodiment of a method for planning a procedure for contrast imaging of a patient. The method 300 comprises a step 310 of providing a planning image of a region of interest of the subject. Then in step 320 the amount of contrast agent in each voxel of the planning image is estimated based on characteristics of tissue indicated by the voxel and on characteristics of the contrast agent. Moreover, in step 330 the planning image and the estimated amount of contrast agent in each voxel are used for generating an attenuation image, wherein the attenuation image is indicative of the attenuation caused by the contrast agent for each voxel in the region of interest. In step 340, finally, a procedure for contrast imaging of the patient is planned based on the attenuation image.

Although in above described embodiments imaging settings for the contrast imaging procedure are planned, in other embodiments, alternatively or additionally also an administration protocol for a contrast agent can be planned. In this case, for instance, an imaging setting can be provided and a default administration protocol or a default contrast agent dose can be varied until a preferred contrast between the tissue of interest and/or a predetermined tissue is reached. Also both the imaging settings and the administration protocol and/or administration dose can be varied at the same time.

When performing patient and/or organ specific dose planning for a patient for contrast imaging, i.e. diagnostic imaging, a contrast agent will not be present in the planning image, i.e. a 3D scout scan. Thus, tube current modulation or other types of sophisticated dose modulation techniques will be limited in effectiveness as well as any kind of prospective dose planning. Ideally the amount of contrast agent present in the final, diagnostic image would be known in advance to perform the exact planning calculations. This invention overcomes this problem by, *a priori,* estimating the amount of contrast agent being present in the different regions of a region of interest to be imaged using a planning image, i.e. 3D scout scan, acquired using, for instance, a CT system that can perform ultra-low-dose acquisition of a preview scan, via angular sparse sampling in a CT system equipped with a grid switching x-ray tube.

In this invention, first the planning image, i.e. a 3D scout scan image, is acquired and reconstructed. Then, based on the planning image, i.e. the 3D scout scan image, a segmentation is performed of the region of interest in the planning image. An anatomical segmentation method is used that identifies regions in the planning image that will have augmented contrast during the final diagnostic acquisition, i.e. during contrast imaging, for instance, a contrasted CT acquisition. Further, the amount of contrast agent to be expected in certain anatomical regions identified by the segmentation method is estimated using application or protocol specific, *a priori,* knowledge of the contrast agent. The planning image, i.e. 3D scout image, is then augmented by adding the expected additional attenuation as can be expected after applying the contrast agent to the patient during the diagnostic imaging.

The planning image, i.e. 3D scout scan, can be acquired, for instance, using sparse angular sampling with 150 views per turn to avoid photo starvation at an ultra-low-dose setting. Such a sparse angular sampling is possible, for instance, when using a CT system equipped with a grid switching x-ray tube.

Based on the planning image a segmentation is performed, wherein the segmentation can be used to segment organs or any other kinds of anatomical regions. The segmentation can be performed using atlas and/or organ models. The result of the segmentation is then a 3D map that gives for each voxel and organ/region a probability that the voxel belongs to an organ or region. In one preferred embodiment the probabilities can only be 0 or 1 such that a voxel can only belong or not belong to a specific segment. Alternatively, also other probabilities can be used. Based on the segmented 3D map a certain expected amount of contrast agent being present in each voxel is then determined. The expected amount can be derived, for instance, from a database of clinical examples of contrasted acquisitions. It can depend on the application and type of contrast agent that should be used. Moreover, the model used for segmentation can already contain labels with specified parts that will contain a certain amount of contrast agent given a specific application protocol. From the expected amount of contrast agent the expected additional attenuation caused by the contrast agent in a diagnostic image can be determined. The planning image, i.e. 3D scout scan image, is then augmented by adding the expected additional attenuation that can be expected after applying the contrast agent to the patient during the diagnostic imaging. The resulting attenuation image is then used for any kind of calculation of planning the administration of the contrast agent or of calculation of individual tube current modulation patterns or dose maps for prospective dose planning.

Other variations to the disclosed embodiments can be understood and effected by those skilled in the art and practicing the planned invention from the study of the drawings, the disclosure and the appendant claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like the providing of a planning image or the estimating of an amount of contrast agent performed by one or several units or devices can be performed by any other number of units or devices. The procedures and/or the operation of the system can be implemented as program code means of a computer program and/or as dedicated hardware.

A computer program may be stored/distributed in a suitable medium, such as any optical storage medium or a solid state medium, supplied together with or as part of other hardware, but might also be distributed in other forms, such as via the internet or other wired or wireless telecommunication systems.

Any reference signs in the claims should not be construed as limiting the scope.

The invention refers to a system for planning a procedure for contrast imaging. A contrast agent estimation unit estimates an amount of contrast agent, which would be present in tissue represented by a voxel of a planning image, for instance a CT image, during contrast imaging, based on characteristics of the tissue and the contrast agent. An attenuation image generating unit generates an attenuation image based on the planning image and the estimated amount of contrast agent, and a procedure planning unit plans the procedure based on the attenuation image. Thus, the planning of the procedure can be based on the attenuation image showing for each patient an expected image resulting from contrast imaging. Accordingly, the planning of the administration of the contrast agent and/or the imaging settings, can be based on reliable and detailed data such that the planning for an individual patient becomes more effective.

## Claims

1. A system for planning a procedure for contrast imaging of a patient, wherein the system comprises:
- a planning image providing unit (4) for providing a planning image of a region of interest of the patient,
- a contrast agent estimation unit (6) for estimating for each voxel of the planning image an amount of contrast agent, which would be present in tissue represented by the respective voxel of the planning image during contrast imaging, based on characteristics of the tissue represented by the respective voxel and on characteristics of the contrast agent,
- an attenuation image generating unit (7) for generating an attenuation image based on the planning image and the estimated amount of contrast agent for each voxel, wherein the attenuation image is indicative of the attenuation caused by the contrast agent in the region of interest during contrast imaging,
- a procedure planning unit (8) for planning a procedure for contrast imaging of the patient based on the attenuation image.

2. The system according to claim 1, wherein the contrast agent estimation unit (6) is further adapted to estimate the amount of contrast agent, which would be present in tissue represented by the respective voxel of the planning image during contrast imaging, based on a predetermined administration protocol of the contrast agent and/or a predetermined imaging setting of an imaging unit used for contrast imaging.

3. The system according to one of the preceding claims, wherein the attenuation image generating unit (7) is adapted to determine an estimate of an attenuation caused by the contrast agent in tissue represented by a voxel based on the estimated amount of contrast agent in tissue represented by the voxel, wherein for generating the attenuation image the attenuation image generating unit (7) is adapted to add the estimate of the attenuation caused by the contrast agent for each voxel to the planning image.

4. The system according to one of the preceding claims, wherein the attenuation image generating unit (7) is adapted to determine the estimate of an attenuation caused by the contrast agent for a voxel based on a predetermined linear relationship between an amount of contrast agent in tissue and the attenuation caused by the contrast agent.

5. The system according to one of the preceding claims, wherein the attenuation image generating unit (7) is adapted to determine the estimate of an attenuation caused by the contrast agent with respect to radiation energy provided by an imaging unit planned to be used during contrast imaging or with respect to contributions of photon and Compton scattering to the attenuation.

6. The system according to one of the preceding claims, wherein the system further comprises a segmentation providing unit (5) for providing a segmentation of the planning image into a plurality of segments, wherein the contrast agent estimation unit (6) estimates the amount of contrast agent present in tissue represented by a voxel belonging to a segment based on characteristics of tissue within the segments.

7. The system according to claim 6, wherein the segmentation unit (5) is adapted to determine a probability that a voxel of the planning image represents a specific organ or a specific anatomical region, wherein the segmentation unit (5) is further adapted to provide the segmentation of the planning image based on the probability.

8. The system according to one of the claims 6-7, wherein the segmentation unit (5) is adapted to segment the planning image based on a predetermined anatomical atlas or based on a predetermined organ model.

9. The system according to one of the preceding claims, wherein the procedure planning unit (8) is adapted for planning imaging settings of an imaging unit planned to be used during the procedure for contrast imaging based on the attenuation image.

10. The system according to one of the preceding claims, wherein the planning image provided by the planning image providing unit (4) has been acquired with the same imaging unit planned to be used during the procedure for contrast imaging.

11. The system according to one of the preceding claims, wherein the planning image is a 3D scout scan image.

12. The system according to one of the preceding claims, wherein the planning image provided by the planning image providing unit (4) has been acquired using a CT-system (41) with a grid-switching x-ray tube for providing the planning image.

13. The system according to one of the preceding claims, wherein the planning image provided by the planning image providing unit (4) has been acquired using a sparse angular sampling acquisition.

14. A method for planning a procedure for contrast imaging of a patient, the method comprising the steps of:
- providing (310) a planning image of a region of interest of the patient,
- estimating (320) for each voxel of the planning image an amount of contrast agent, which would be present in tissue represented by the respective voxel of the planning image during contrast imaging, based on characteristics of the tissue represented by the respective voxel and on characteristics of the contrast agent,
- generating (330) an attenuation image based on the planning image and the estimated amount of contrast agent for each voxel, wherein the attenuation image is indicative of the attenuation caused by the contrast agent in the region of interest during contrast imaging,
- planning (340) a procedure for contrast imaging of the patient based on the attenuation image.

15. A computer program for planning a procedure for contrast imaging of a patient, the computer program comprising program code means for causing the system of any of claims 1-13 to carry out the steps of the method as defined in claim 14 when the computer program is run on a computer controlling the system.
